# EUROPEAN PATENT APPLICATION

(11) **EP 4 488 659 A1**
(43) Date of publication of application: **08.01.2025**
(21) Application number: 23183799.8
(22) Date of filing: 06.07.2023
(51) Int. Cl.: G01N 21/31, G01N 21/85, G01N 21/94, G01N 33/00, G01N 21/33, G01N 21/3504, G01N 21/15, G01M 15/10

(54) **EQUIPMENT AND PROCEDURE FOR CONTINUOUS MEASUREMENT OF POLLUTANT EMISSIONS IN THE EXHAUST SYSTEM OF MOTOR VEHICLES**

(71) Applicant: Opus RS Europe, S.L., 28015 Madrid (ES)
(72) Inventor: de la Fuente Egido, Josefina, 28015 Madrid (ES)
(74) Representative: Lahidalga de Careaga, Jose Luis

(57) **Abstract**

Equipment configured to be installed in a final section of an exhaust pipe of a motor vehicle for continuous measurement of pollutant emissions in the exhaust gas of the motor vehicle is disclosed. The equipment comprises

- an outer cylinder (1) having a diameter equal to that of the exhaust pipe;

- an inner cylinder (2) concentric to the outer cylinder and joined therewith by bearings, the cylinders being rotatable relative to each other between a working position and a rest position;

- three light emitters (3, 4, 5) presented on an inner surface of the outer cylinder (1);

- a small box containing a data receiving device (20), a data transmitter device (21), and an emission detector device (22) arranged on an outer surface of the outer cylinder (1);

- three windows (9, 10, 11) arranged on an outer surface of the inner cylinder (2);

- three light receivers (6, 7, 8) arranged on an inner surface of the inner cylinder (2) and opposite the light emitters (3, 4, 5) when the cylinders are in the working position;

- three shutters (12, 13, 14) arranged on the outer surface of the inner cylinder (2), wherein the shutters are configured to obstruct the light from the light emitters (3, 4, 5) when the cylinders are in the rest position; and

a rotation device (24) for rotating the cylinders arranged on the inner surface of the inner cylinder (2).

## Description

The present invention relates to an equipment and procedure for continuous measurement of pollutant emissions in the exhaust system of motor vehicles. It is therefore a matter of measuring the pollutant emissions of vehicles continuously as they move and throughout the natural life of the vehicle based on photonic technologies.

In this way, important information on pollutant emissions is collected and sent to the place where it is needed, in real time and its evolution according to all external and internal elements.

### TECHNICAL FIELD

This invention relates to the automotive industry. To be more specific, this invention pertains to the measurement and control of pollutant gases.

### BACKGROUND OF THE INVENTION

Current technology has effective pollutant emission meters, especially in devices across the width of a roadway emitted by motor vehicles.

There are old and modern means such as those that work by laser spectroscopy by means of light intensity absorption devices that work on the actual road from side to side of the same, measuring the pollutant emissions emitted by motor vehicles, through the reflection of light or and with information collected from the same place as the emitters by sensors located on one side of the road.

Among the antecedents found, reference can be made to the following, some made by the same inventors as this patent application.

The present invention refers to continuous pollutant emission measurement equipment located in the exhaust system of motor vehicles. It is therefore a matter of measuring the pollutant emissions of vehicles continuously as they move and throughout the natural life of the vehicle.

US Patent US4924095, uses multiple beam paths to sample a cross-sectional "slice" of the exhaust plume. The volume of the slice is determined and used to calculate an absolute concentration of one or more exhaust components. Such a system proved to be inaccurate and unfeasible in practice due to the irregular dispersion of the exhaust plume and significant difFiculties in calculating the volume of the exhaust plume.

As a more modern element we can refer to US 5877862 which discloses a system for analyzing exhaust gases using tunable infrared spectroscopy. A laser beam is directed through an exhaust plume, reflected by a reflector and then received by a detector after a second pass through the exhaust plume.

But efFectively these are spot measurements, notcontinuous or individually particularized over the lifetime of the vehicle.

The inventor has not found any remarkable precedent that could be considered as a direct antecedent.

### DESCRIPTION OF THE INVENTION

The present invention relates to a miniaturized equipment based on photonic technologies for the measurement of vehicle pollution emissions.

It is based on emitters and receivers of radiation in the infrared and ultraviolet bands.

Its working position is in the final section of the exhaust pipe of vehicles. These emitters and receivers are tunable in wavelength.

The equipment is designed to continuously measure the amount of particulate matter and other gases (CO, HC, NOX and CO2) emitted by vehicles in circulation.

The vehicle pollution equipment consists of a tubular structure with a circular cross section, which is made up of two parts that are tightly joined and that allow the closing and opening of the same by means of a rotation system.

This opening and closing system ensures that the sensor remains closed and protected from dirt when not in use and opens automatically when the gas emitted by the vehicle circulates over it.

Inside the tubular structure are wavelength-tunable infrared emitters and receivers, which are placed in opposite positions along the length of the tube.

The emitters emit a beam of infrared Y uv light that passes through the tube and is picked up by the receivers, which are located in the opposite position.

The infrared Y uv light is modulated at a specific frequency, so that when contaminating particles are in the beam, they absorb part of the modulated light.

Receivers pick up the modulated signal and send it to a signal processing system, which interprets the information received and determines the amount of particles and gases present in the light beam.

The signal processing device is designed to analyze the modulated signal at various frequencies, allowing different types of compounds to be identified and measured.

In summary, the vehicle pollution sensor based on wavelength-tunable mini emitters and receivers is an innovative and efficient solution for measuring the amount of particulate pollutants emitted by vehicles in circulation.

Its tubular design with an opening and closing system protects the sensor from dirt and its wavelength-tunable infrared and UV emitters and receivers technology allows accurate and efficient measurement of different types of gases.

This invention has a great potential for applications in the field of measurement and control of air pollution in urban areas and roads.

### DESCRIPTION OF THE DRAWINGS

In order to complement the description being made and with the object of aiding a better understanding of the characteristics of the invention, in accordance with a preferred example of the practical realization thereof, there is attached as an integral part of said description, a set of drawings wherein, with illustrative and non-limiting character, the following has been represented:

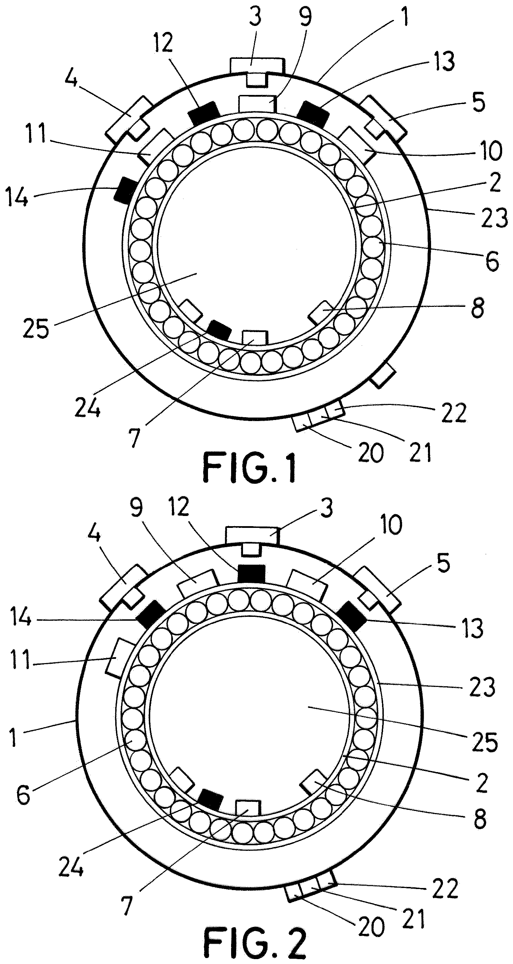

Figure 1. Shows a schematic view of the central section of the measuring equipment in working position.
FIGURE 2. Shows a schematic view of the central section of the measuring equipment in rest position.

And in these figures the same elements are identified with identical numbering:
(1).- outer cylinder,
(2). - inner cylinder
(3), (4), (5).- light emitters,
(6), (7), (8).- light receivers.
(9), (10), (11).- windows,
(12), (13), (14).- light cleaners/obstructors,
(20).- data receivers,
(21).- data transmitter,
(22).- emission detector/processor,
(23).- slewing bearings,
(24).- turning device,
(25).- light emissions

### PREFERRED EMBODIMENT OF THE INVENTION

The present invention relates to an equipment and procedure for continuous measurement of pollutant emissions located in the exhaust system of motor vehicles.

It is therefore a matter of measuring the pollutant emissions of vehicles continuously as they move and throughout the natural life of the vehicle based on photonic technologies and more specifically on emissions and receptions of radiation in the infrared and ultraviolet bands.

In this way, important information on pollutant emissions is collected and sent to the place where it is needed, in real time and its evolution according to all external and internal elements.

It is based on emitters and receivers of radiation in the infrared and ultraviolet bands.

The device is located in the final section of the exhaust pipe of vehicles and is made up of the following elements:
1. Outer cylinder (1) of diameter equal to that of the exhaust pipe where it is installed as part of the exhaust pipe.
   This cylinder has three light emitters, (3), (4) and (5), separated by at least 60º and with the light emitter focused on the cylinder axis.
   These emitters are designed to emit infrared and ultraviolet light (25) and are tunable in wavelength.
   On the outer surface of the cylinder (1) there is a small box containing the data receiver device (20), the data emitter device (21) and the emission detector device (22), necessary to detect when the emission of gases begins and to send the rotation device (24) the rotation order to obtain the alignment of emitter and receiver.
2. Inner cylinder (2), concentric to the previous one (1) and of smaller diameter, articulated with it by bearings (23) at its ends.
   On the outer surface of the cylinder (2) there are three windows (9), (10) and (11) to let the light (25) sent by the emitters (3), (4), (5) and received by the receivers (6), (7), (8) located homothetically with the emitters on the inner surface of the cylinder (2).
   On the outer surface of the cylinder (2) there are three shutters / wipers, (12), (13), (14) that obstruct the passage of light (25) when the vehicle is at rest and also when positioned or removed to allow the passage of light, function as wipers of the emitters, by friction.

The system is designed to continuously measure the amount of particles and other gases (CO, HC, NOX and CO2) emitted by vehicles in circulation.

The emitters (3), (4), (5) emit a beam of infrared light (Y,uv) that passes through the tube and is captured by the receivers (6), (7), (8) which are located in the opposite position. The infrared light (Y, uv) is modulated at a specific frequency, so that when the contaminating particles are in the light beam, they absorb part of the modulated light. The receivers pick up the modulated signal and send it to the data receiver (20) which interprets the information received and determines the amount of particles and gases present in the light beam. The data processing device (22) is designed to analyze the modulated signal at various frequencies, making it possible to identify and measure different types of compounds and send the information in real time to the appropriate data processing center.

Having sufFiciently described the nature of the invention, as well as the manner of putting it into practice, it should be noted that the provisions indicated above are susceptible to modifications of detail insofar as they do not alter its fundamental principles, set forth in the preceding paragraphs and summarized in the following claims.

## Claims

1. Equipment for the continuous measurement of pollutant emissions located in the exhaust system of a motor vehicle by means of photonic technologies **characterized by** being constituted by two concentric cylinders (1) and (2) that rotate between them by means of bearings (23) at their ends and where the outer cylinder (1) is of diameter equal to that of the exhaust pipe where it is installed and where on its inner face it has three light emitters, (3), (4) and (5), separated by at least 60° and where on the outer surface of the cylinder (1) there is a small box containing a data receiving device (20), a data transmitter device (21) and an emission detector device (22) and where the inner cylinder (2), concentric to the previous outer cylinder (1) and of smaller diameter and where on the outer surface of the inner cylinder (2) there are three windows (9), (10) and (11) to let in the light (25) sent by the transmitters (3), (4), (5) and received by the emitters (3), (4), (5), (6), (7), (8), (8), (9), (9), (9), (10), (10) and (11), (5) and the receivers (6), (7), (8) located homothetically with the emitters on the inner surface of the inner cylinder (2) and where on the outer surface of the inner cylinder (2) are located three shutters / wipers, (12), (13), (14), and where on the inner surface of the inner cylinder (2) is located a turning device for the cylinders (1) and (2).

2. Procedure for continuous measurement of pollutant emissions located in the exhaust system of motor vehicles by means of photonic technologies and **characterized by** comprising the following steps:
light emitters, (3), (4) and (5) emit a beam of infrared light (Y,uv) which passes through the tube and is captured by receivers (6), (7), (8) which are located in the opposite position
the infrared light (Y, uv) is modulated at a specific frequency depending on the contaminants to be detected, so that when the contaminant particles are in the light beam, they absorb part of the modulated light
the receivers pick up the modulated signal and send it to a data receiver (20) which interprets the information received and determines the amount of particles and gases present in the light beam
a data processing device (22) analyzes the modulated signal at various frequencies,
identifies and measures different types of compounds and sends the information in real time to a data processing center.

3. Procedure for continuous measurement of pollutant emissions located in the exhaust system of motor vehicles by means of photonic technologies according to claim 2 and **characterized in that** the three light emitters, (3), (4) and (5) are designed to emit infrared and ultraviolet light (25) and are tunable in wavelength.
